# EUROPEAN PATENT SPECIFICATION

(11) **EP 2 445 533 B1**
(45) Date of publication and mention of the grant of the patent: **08.02.2017**
(21) Application number: 09846372.2
(22) Date of filing: 26.06.2009
(51) Int. Cl.: A61K 45/06, A61K 31/19, A61K 31/192, A61K 31/16, A61K 38/15, A61P 17/00

(54) **METHOD FOR TREATING OR AMELIORATING MUCOCUTANEOUS OR OCULAR TOXICITIES**
VERFAHREN ZUR BEHANDLUNG BZW. LINDERUNG VON SCHLEIMHAUT- ODER AUGENTOXIZITÄTEN
MÉTHODE POUR TRAITEMENT OU AMÉLIORATION DE TOXICITÉS MUCOCUTANÉES OU OCULAIRES

(43) Date of publication of application: 02.05.2012
(73) Proprietor: Sunny Pharmtech Inc.,, New Taipei City (TW)
(72) Inventor: PUI, Nam-Mew, Taiwan (CN); CHUNG, Yih-Lin, Taiwan (CN)
(74) Representative: Fuchs Patentanwälte Partnerschaft mbB
(86) International application number: PCT/CN2009/072474
(87) International publication number: WO 2010/148572

(56) References cited:
- EP-A1- 1 574 213
- WO-A2-00/08048
- WO-A2-2006/113479
- CN-A- 1 414 971
- CN-A- 1 933 802
- CN-A- 101 152 179
- CN-A- 101 175 492
- CN-A- 101 222 938
- CN-A- 101 280 317
- US-A1- 2006 275 370
- US-A1- 2008 107 646
- US-A1- 2008 182 865
- US-A1- 2008 207 724
- US-A1- 2009 149 511
- PEREZ-SOLER ROMÁN ET AL: "HER1/EGFR inhibitor-associated rash: future directions for management and investigation outcomes from the HER1/EGFR inhibitor rash management forum", THE ONCOLOGIST, ALPHAMED PRESS, US, vol. 10, no. 5, May 2005 (2005-05), pages 345-356, XP002451871, ISSN: 1083-7159, DOI: 10.1634/THEONCOLOGIST.10-5-345
- MELOSKY B ET AL: "Management of skin rash during EGFR-targeted monoclonal antibody treatment for gastrointestinal malignancies: Canadian recommendations", CURRENT ONCOLOGY, ELSEVIER, NEW YORK, NY, US, vol. 16, no. 1, January 2009 (2009-01), pages 14-24, XP002674583, ISSN: 0743-930X
- GALIMONT-COLLEN A F S ET AL: "Classification and management of skin, hair, nail and mucosal side-effects of epidermal growth factor receptor (EGFR) inhibitors", EUROPEAN JOURNAL OF CANCER, PERGAMON PRESS, OXFORD, GB, vol. 43, no. 5, March 2007 (2007-03), pages 845-851, XP027016596, ISSN: 0959-8049 [retrieved on 2007-03-16]
- MASCIA F ET AL: "Blockade of the EGF receptor induces a deranged chemokine expression in keratinocytes leading to enhanced skin inflammation", AMERICAN JOURNAL OF PATHOLOGY; [10640], ELSEVIER INC, US, vol. 163, no. 1, July 2003 (2003-07), pages 303-312, XP002410413, ISSN: 0002-9440
- MONNERET ET AL: "Histone deacetylase inhibitors", EUROPEAN JOURNAL OF MEDICINAL CHEMISTRY, EDITIONS SCIENTIFIQUE ELSEVIER, PARIS, FR, vol. 40, no. 1, January 2005 (2005-01), pages 1-13, XP027857598, ISSN: 0223-5234 [retrieved on 2005-01-01]

## Description

### Field of the Invention

The present invention relates to treatment of skin disorders, and in particular relates to using an HDAC inhibitor to treat mucocutaneous or ocular toxicities.

### Description of the Related Art

With the advances in understanding of aberrant signaling pathways in various diseases or disorders such as cancer, inflammatory disease, immunological disorder and degenerative disease, many pivotal regulators of cell behavior, response, growth, survival and apoptosis have emerged as candidates, such as EGFR, VEGF, tyrosine kinases, serine/threonine kinases, and MAPK/ERK for molecular target-based therapy (Lacouture ME. Nat Rev Cancer 6:803-812, 2006). Experimentation due to the above, has led to the development of innovative molecular targeting techniques including monoclonal antibodies, small chemical molecules, peptide mimetics and antisense oligonucleotides. However, coincident inhibition of the EGFR receptor, tyrosine kinase activity or MAPK/ERK pathways in tissues that depend on signaling for normal function has undesirable consequences (Perez-Soler R, et al. J Clin Oncol 23:5235-5246, 2005). Because of the essential functions of EGFR, tyrosine kinase and MAPK/ERK signaling in the skin, mucosa and cornea, undesirable side effects to the mucocutaneous and ocular sites commonly occur. Inhibition of EGFR, tyrosine kinase activity or MAPK/ERK pathways has been shown to reduce DNA synthesis, arrest cell growth, and induce premature differentiation in keratinocytes (Miettinen PJ, et al. Nature 376:337-341, 1995). Pharmacologically mediated blockade of EGFR results in growth arrest and apoptosis in cells that are dependent on EGFR for survival, through the inhibition of downstream pathways such as the MAPK pathway, the PI3K (phosphatidylinositol 3-kinase)-Akt pathway, the stress-activated protein kinase pathway that involves protein kinase C, and the Janus kinase (Jak)-STAT (signal transducer and activator of transcription) pathway. As a result of inhibition of EGFR that is primarily expressed in undifferentiated, proliferating keratinocytes in the basal and suprabasal layers of the epidermis and the outer layers of the hair follicle, the epidermis layer become thinner, which impairs barrier function and further sensitizes the epithelial cells to UV light and radiation. On the other hand, enhanced EGFR activation is associated with increased CXCL8 expression, and diminished CCL2, CCL5, and CXCLIO expression (Mascia F, et al. Am J Pathol 163:303-312, 2003). In contrast, an opposite pattern is seen for impairment of EGFR activation. In experimentation using mouse, skin application of a selective EGFR tyrosine kinase inhibitor led to more severe contact hypersensitivity responses, with increased epidermal levels of CCL2, CCL5, and CXCLIO, and a higher number of monocytes/macrophages and T lymphocytes in the skin. The findings suggested that EGFR modulates skin reaction by affecting chemokine expression in keratinocytes.

EGFR governs the homeostatic maintenance, repair and reaction of epithelial tissues. Thus, many targeted therapy drugs interfering with EGFR or MAPK/ERK signaling pathways or tyrosine activity can cause skin changes including rash, dry skin, xerosis, itching, red sore cuticles, paronychia, hand foot reaction or syndrome, telangiectasia, and changes in hair growth or skin color (Galimont-Collen AFS, et al. Eur J Cancer 43:845-851, 2007). The changes are normal body response side effects due to the targeted therapy drug and not signs of a drug allergy. The side effects may develop into chronic eczema and increase the risk of secondary infections. A painful sensitivity of the hands and feet is the earliest symptom. Then, redness and swelling starts in the palms of the hands and the soles of the feet. The most common skin change is a papulopustular rash. It often looks a lot like acne and shows up on the scalp, face, chest, and upper back. In severe cases it can affect other parts of the body, and the blisters can open up and become sores. The rash is often worst within the first few weeks of molecular targeted treatment. By about the 4th week of a molecular targeted treatment, the skin usually crusts and gets very dry and red. In the weeks after that, round, flat or raised red spots and "whitehead" pimples with pus in the center often appear. The rash can be itchy. The cause of hand foot reaction or syndrome is not conclusively known. It may have to do with damage to the tiny blood vessels in the hands and feet, or with the drugs themselves leaking out of the blood vessels and causing tissue damage. Other less common side effects caused by targeted therapies have also been found in the mucosa and eye. They include diarrhea, nausea, vomiting, mouth sores, cough, and in particular, trichomegaly of the eyelashes, which can scratch the eye and may be accompanied by conjunctivitis and other ocular disorders that can lead to significant discomfort and blurred vision.

The mucocutaneous or ocular toxicities or side effects resulting from the molecular targeted therapy can be painful, cause physical and psycho-social discomfort, and affect the abilities for patients to walk, eat, and carry out normal activities. The targeted treatment discontinuation or delay due to the mucocutaneous or ocular toxicities occurs in many patients. Thus, there is a need for a novel method to treat or ameliorate the toxicities.

### Brief Summary of the Invention

Described herein is a pharmaceutical composition for use in treatment of mucocutaneous, ocular toxicities, or side effects resulting from the blocking of cell growth and survival signal transduction pathways, comprising a therapeutically effective amount of a histone deacetylase (HDAC) inhibitor and/or a pharmaceutically acceptable salt or solvate thereof.

Described herein is a pharmaceutical composition for use in diminishing the dermatological or epithelial side effects of a papulopustular rash, dry skin, itching, dermatitis, hand foot syndrome, mucositis, loss of hair, increased growth of the eyelashes and facial hair, brittle deformed nails and periungual swelling resulting from the molecular therapy targeting the epidermal growth factor receptor (EGFR), MAPK(mitogen-activated protein kinase)/ERK (extracellular regulated kinase), vascular endothelial growth factor (VEGF) receptor, or PI3K (phosphatidylinositol 3-kinase)-Akt-mTOR pathways with or without combination of chemotherapy and/or radiotherapy in a subject in need thereof. The pharmaceutical composition is used for topically applying to an affected skin or mucosal area of the subject a composition comprising an effective amount of an HDAC inhibitor, or a hyperacetylating agent selected from the group consisting of valproic acid, phenylbutyrate, or trichostatin A, and mixtures thereof and a suitable salt and/or a suitable carrier.The HDAC inhibitor or the hyperacetylating agent is administered to the patient prior to molecular targeted therapy, during molecular targeted therapy, and/or after molecular targeted therapy.

The invention provides a pharmaceutical composition comprising a therapeutically effective amount of a histone deacetylase (HDAC) inhibitor and/or a pharmaceutically acceptable salt or solvate thereof for use in treating mucocutaneous or ocular toxicities, or side effects resulting from blocking of cell growth and survival signal transduction pathways caused by an EGFR inhibitor, wherein.

The HDAC inhibitor is valproic acid, phenylbutyrate or trichostatin A.

In one embodiment the pharmaceutical composition is non-orally administered.

In one embodiment the pharmaceutical composition is present in an amount of the HDAC inhibitor from 0.00001% to 100% by weight of the pharmaceutical composition.

In one embodiment the pharmaceutical composition is a cream, an ointment, a gel, a paste, a powder, an aqueous solution, a spray, a suspension, a dispersion, a slave, a lotion, a patch, a suppository, a liposome formation, a mouth wash, an enema, an injection solution, an eye drop, an ear drop, a drip infusion, a microcapsule, a nanocapsule, an occlusive skin conditioning agent, a biocompatible polymer, or an agent to prolong retention and sustain action of the HDAC inhibitor in the tissue.

In one embodiment, the pharmaceutical composition further comprises a penetration enhancing agent, or a pH adjusting agent to provide a pH in the range of approximately 3.0 to 13.0.

In one embodiment the mucocutaneous or ocular toxicities, or side effects comprise dermatological or epithelial side effects including papulopustular rash, dry skin, itching, dermatitis, hand foot syndrome, mucositis, loss of hair, increased growth of the eyelashes and facial hair, and/or brittle deformed nails and periungual swelling.

Described herein is the use of a composition comprising an effective amount of an HDAC inhibitor, or a hyperacetylating agent selected from the group consisting of valproic acid, phenylbutyrate, trichostatin Aand mixtures thereof and a suitable salt and/or a suitable carrier in the manufacture of a medicament for diminishing the dermatological or epithelial side effects of a papulopustular rash, dry skin, itching, dermatitis, hand foot syndrome, mucositis, loss of hair, increased growth of the eyelashes and facial hair, brittle deformed nails and periungual swelling resulting from the molecular therapy targeting the epidermal growth factor receptor (EGFR), MAPK(mitogen-activated protein kinase)/ERK (extracellular regulated kinase), vascular endothelial growth factor (VEGF) receptor, or PI3K (phosphatidylinositol 3-kinase)-Akt-mTOR pathways with or without combination of chemotherapy and/or radiotherapy.

Described herein is a combination comprising an HDAC inhibitor and/or a pharmaceutically acceptable salt or solvate thereof, and an EGFR inhibitor. Preferably, the combination is a form of a pharmaceutical composition comprising an HDAC inhibitor and/or a pharmaceutically acceptable salt or solvate thereof, and an EGFR inhibitor. Preferably, the HDAC inhibitor and the EGFR inhibitor are concurrently administered to the subject in the same formulation and the same route, or sequentially in a different formulation and/or a different route.

Described herein is a method for treating diseases or disorders by blocking EGFR, tyrosine kinase activation, or MAPK/ERK pathways or any of the downstream biological effects of EGFR or tyrosine kinase activation without resulting in mucocutaneous, ocular toxicities, or side effects, comprising administration of a combination comprising an HDAC inhibitor and/or a pharmaceutically acceptable salt or solvate thereof, and an EGFR inhibitor. The relevant diseases or disorders are selected from cancer, inflammatory disease, immunological disorder and degenerative disease. The mucocutaneous, ocular toxicities, or side effects include dermatological or epithelial side effects of papulopustular rash, dry skin, itching, dermatitis, hand foot syndrome, mucositis, loss of hair, increased growth of the eyelashes and facial hair, and/or brittle deformed nails and periungual swelling.

Described herein is a use of a pharmaceutical composition comprising an HDAC inhibitor and/or a pharmaceutically acceptable salt or solvate thereof, and an EGFR inhibitor in the manufacture of a medicament for treating diseases or disorders by blocking EGFR, tyrosine kinase activation, or MAPK/ERK pathways or any of the downstream biological effects of EGFR or tyrosine kinase activation without resulting in mucocutaneous, ocular toxicities, or side effects. The relevant diseases or disorders are selected from cancer, inflammatory disease, immunological disorder and degenerative disease. The mucocutaneous, ocular toxicities, or side effects include dermatological or epithelial side effects of papulopustular rash, dry skin, itching, dermatitis, hand foot syndrome, mucositis, loss of hair, increased growth of the eyelashes and facial hair, and/or brittle deformed nails and periungual swelling.

A detailed description is given in the following embodiments with reference to the accompanying drawings.

### Brief Description of the Drawings

The present invention can be more fully understood by reading the subsequent detailed description and examples with references made to the accompanying drawing, wherein:
Fig. 1 shows that different HDAC inhibitors affect the effects of the EGFR inhibitor on the expression of the chemokine that is associated with skin side effects;
Fig. 2 shows that treatment with the topical HDAC inhibitor can ameliorate the mouse skin swelling augmented by inhibition of EGFR. The Student's t test was used to determinate the statistical difference (*P<0.05) between the placebo gel and the 2.5% phenylbutyrate gel. One-way ANOVA followed by the Dunnett's test was used to compare the values with the EGFR inhibition. ^P< 0.05 was considered significant vs. PD168393 (i.e., A-[(3-Bromophenyl)amino]-6-acrylamidoquinazoline, an EGFR inhibitor); and
Fig. 3 shows comparison of histology (H&E) of representative examples of the EGFR inhibitor (PD168393)-augmented skin reaction to DNFB irritation at 48 hours in the ears of mice treated with or without the placebo gel or 2.5% phenylbutyrate gel.

The following description is of the best-contemplated mode of carrying out the invention.

Inhibition of EGFR, tyrosine kinases or MAPK/ERK pathways at mucocutaneous or ocular tissues can result in abnormal proliferation, migration, differentiation and apoptosis of target and epithelial cells, and consequent disruption of the integrity of the skin, mucosa and cornea with the subsequent recruitment of inflammatory cells. Thus, the localization of the side effects to the mucocutaneous or ocular sites and the high frequency of the reactions are thought to reflect the function of EGFR, tyrosine kinases and MAPK/ERK pathways in the epidermis, hair follicle, periungual, mucosa, and ocular tissues.

The invention demonstrates that administration of an HDAC inhibitor formulated in a suitable carrier is effective at ameliorating the skin reaction abnormally augmented by the EGFR inhibitor. Thus, HDAC inhibitors can advantageously be used to treat the skin toxicities otherwise caused by use of an EGFR, tyrosine kinase or MAPK/ERK signaling inhibitor. Accordingly, the present invention provides a pharmaceutical composition comprising an HDAC inhibitor in a pharmaceutically acceptable carrier for use in treating or ameliorating mucocutaneous, ocular toxicities, or side effects resulting from blocking of cell growth and survival signal transduction pathways.

As used herein, the term "EGFR, tyrosine kinase or MAPK/ERK inhibitor" refers to any EGFR, tyrosine kinase or MAPK/ERK inhibitor that is currently known in the art and includes any chemical entity that, upon administration to a subject, results in inhibition of a biological activity associated with activation of the EGFRs, tyrosine kinases or MAPK/ERK pathways in the subject, including any of the downstream biological effects otherwise resulting from the binding to an EGFR of its natural ligand. Such EGFR, tyrosine kinase or MAPK/ERK inhibitors include any agent that can block EGFR, tyrosine kinase activation, or MAPK/ERK pathways or any of the downstream biological effects of EGFR or tyrosine kinase activation that are relevant to treating a cancer, inflammatory disease, immunological disorder or degenerative disease in a subject. Such an inhibitor can act by binding directly to the extracellular or intracellular domain of the growth factor receptor, inhibiting its associated tyrosine kinase activity, or interfering with the related downstream signaling transduction cascade via serine/threonine activity. Alternatively, such the tyrosine kinase inhibitor can act by inhibiting the downstream signal transducers with tyrosine kinase activity.

The term "tyrosine kinase inhibitor" refers to natural or synthetic agents that are enabled to inhibit or block tyrosine kinases, receptor tyrosine kinases or ATP binding sites of the kinases. The so-called EGFR, tyrosine kinase or MAPK/ERK inhibitors include but are not limited to low molecular weight inhibitors, antibodies or antibody fragments, antisense (DNA or RNA) constructs, peptide mimetics and ribozymes.

The HDAC inhibitors activate and repress a subset of genes by remodeling the chromatin structure thereof via the altered status in histone acetylation (Marks et al, J. Natl. Cancer Inst., 92: 1210-1216, 2000; Kramer et al, Trends Endocrinol. Metab., 12: 294-300, 2001). Histone hyperacetylation results in the up-regulation of cell-cycle inhibitors (p21Cip1, p27Kip1, and p16INK4), the down-regulation of oncogenes (Myc and Bcl-2), the repression of inflammatory cytokines (interleukin (IL)-1, IL-8, TNF-. alpha., and TGF-. beta.), or no change (GAPDH and gamma. -actin)(Lagger et al, EMBO J., 21 : 2672-2681, 2002; Richon et al, Clin. Cancer Res., 8: 662-667, 2002; Richon et al, Proc. Natl. Acad. Sci. USA., 97: 10014-10019, 2000; Van Lint et al, Gene Expr., 5: 245-243,1996; Huang et al, Cytokine, 9: 27-36, 1997; Mishra et al, Proc. Natl. Acad. Sci. USA., 98: 2628-2633, 2001; Stockhammer et al, J. Neurosurg., 83: 672-681, 1995; Segain et al, Gut, 47: 397-403, 2000; Leoni et al, Proc. Natl. Acad. Sci. USA, 99: 2995-3000, 2002). In addition to inducing histone hyperacetylation, HDAC inhibitors also induce hyperacetylation of nonhistone proteins such as ribosomal S3, p53 or the Rel-A subunit of NF-.kappa.B, modulate protein kinase C (PKC) activity, inhibit protein isoprenylation, decrease DNA methylation, and bind to nuclear receptors (Webb et al, J. Biol. Chem., 274: 14280-14287, 1999; Chen et al, Science, 293: 1653-1657, 2001). HDAC inhibitors have exhibited properties in inducing cell-cycle arrest, cell differentiation, and apoptotic cell death in tumor cells and in decreasing inflammation and fibrosis in inflammatory diseases (Warrell et al, J. Natl. Cancer Inst., 90: 1621-1625, 1998; Vigushin et al, Clin. Cancer Res., 7: 971-976, 2001; Saunders et al, Cancer Res., 59: 399-404,1999; Gottlicher et al, EMBO J., 20: 6969-6978, 2001; Rombouts et al, Acta Gastroenterol. Belg., 64: 239-246, 2001). Although the effects of HDAC inhibitors induce bulk histone acetylation, they result in apoptotic cell death, terminal differentiation, and growth arrest in tumor cells but no toxicity in normal cells (Garber et al, J. Natl. Cancer Inst., 94: 793-795, 2002). In addition, the modulation of chromatin conformation by the HDAC inhibitors can further radiosensitize tumors whose cells are intrinsically radioresistant, and also sensitize tumor cells to chemotherapy (Ferrandina et al, Oncol. Res., 12: 429-440, 2001; Miller et al, Int. J. Radiat. Biol, 72: 211-218, 1997; Biade et al, Int. J. Radiat. Biol, 77: 1033-1042, 2001).

Active compounds are, in general, histone hyperacetylating agents, such as HDAC inhibitors. Numerous such compounds are known. See, e.g., P. Dulski, Histone Deacetylase as Target for Antiprotozoal Agents, PCT Application WO 97/11366 (Mar. 27, 1997). Examples of such compounds include, but are not limited to:
A. Trichostatin A and its analogues such as: Trichostatin A (TSA); and Trichostatin C (Koghe et al. 1998. Biochem. Pharmacol. 56: 1359-1364) (Trichostatin B has been isolated but not shown to be an HDAC inhibitor).
B. Peptides, such as: Oxamflatin (Kim et al. Oncogene, 18:2461-2470 (1999)); Trapoxin A (TPX) (Kijima et al., J. Biol. Chem. 268, 22429-22435 (1993)); FR901228, Depsipeptide (Nakajima et al., Ex. Cell Res. 241, 126-133 (1998)); FR225497, Cyclic Tetrapeptide (H. Mori et al., PCT Application WO 00/08048 (Feb. 17, 2000)); Apicidin, (Darkin-Rattray et al., Proc. Natl. Acad. Sci. USA 93, 13143-13147 (1996)); Apicidin Ia, Apicidin Ib, Apicidin Ic, Apicidin IIa, and Apicidin IIb (P. Dulski et al., PCT Application WO 97/11366); HC-Toxin (Bosch et al., Plant Cell 7, 1941-1950 (1995)); WF27082 (PCT Application WO 98/48825); and chlamydocin (Bosch et al., supra).
C. Hydroxamic Acid-Based Hybrid Polar Compounds (HPCs), such as: Salicylihydroxamic Acid (SBHA) (Andrews et al., International J. Parasitology 30, 761-768 (2000)); Suberoylanilide Hydroxamic Acid (SAHA) (Richon et al., Proc. Natl. Acad. Sci. USA 95, 3003-3007 (1998)); Azelaic Bishydroxamic Acid (ABHA) (Andrews et al., supra); Azelaic-1-Hydroxamate-9-Anilide (AAHA) (Qiu et al., Mol. Biol. Cell 11, 2069-2083 (2000)); M-Carboxycinnamic Acid Bishydroxamide (CBHA) (Ricon et al., supra); 6-(3-Chlorophenylureido)carpoic Hydroxamic Acid (3-CI-UCHA) (Richon et al., supra); MW2796 (Andrews et al., supra); and MW2996 (Andrews et al., supra). Note that analogs not effective as HDAC Inhibitors are: Hexamethylene bisacetamide (HBMA) (Richon et al. 1998, PNAS, 95:3003-3007); and Diethyl bis(pentamethylene-N,N-dimethylcarboxami- -de) malonate (EMBA) (Richon et al. 1998, PNAS, 95:3003-3007).
D. Short Chain Fatty Acid (SCFA) Compounds, such as: Sodium Butyrate (Cousens et al., J. Biol. Chem. 254, 1716-1723 (1979)); Isovalerate (McBain et al., Biochem. Pharm. 53: 1357-1368 (1997)); Valproic acid; Valerate (McBain et al., supra); 4-Phenylbutyrate (4-PBA) (Lea and Tulsyan, Anticancer Research, 15, 879-873 (1995)); Phenylbutyrate (PB) (Wang et al., Cancer Research, 59, 2766-2799 (1999)); Propionate (McBain et al., supra); Butrymide (Lea and Tulsyan, supra); Isobutyramide (Lea and Tulsyan, supra); Phenylacetate (Lea and Tulsyan, supra); 3-Bromopropionate (Lea and Tulsyan, supra); and Tributyrin (Guan et al., Cancer Research, 60, 749-755 (2000)).
E. Benzamide derivatives, such as: MS-27-275 [N-(2-aminophenyl)-4-[N-(pyridin-3-yl-methoxycarbonyl) aminomethyl]benzamide] (Saito et al., Proc. Natl. Acad. Sci. USA 96, 4592-4597 (1999)); and 3'-amino derivative of MS-27-275 (Saito et al., supra).
F. Other inhibitors, such as: Depudecin [its analogues (mono-MTM-depudecin and depudecin-bisether) do not inhibit HDAC] (Kwon et al. 1998. PNAS 95:3356-3361); and Scriptaid (Su et al. 2000 Cancer Research, 60:3137-3142).

The second compound, combined with the HDAC inhibitor include, but are not limited to, a cytokine, a cytokine inhibitor, an interleukin, an interleukin inhibitor, a growth factor, an angiogenic agent, an angiogenic inhibitor, an anti-neoplastic agent, an anti-inflammatory agent, a steroid, an immunosuppressive agent, a non-steroid anti-inflammation drug, an analgesic agent, an anti-histamine, an anticholinergics, an antipruritic agent, an antibacterial agent, an antiviral agent, an antifungal agent, an antiparasitic agent, an antioxidant agent, retinoic acid, an anti-fibrogenic agent, a vasoactive agent, an adenosine receptor agonist, a vitamin, a leukotriene modifier, an interleukin antagonist, a chemokine antagonist, a mast cell inhibitor, an anti-IgE antibody, a selective serotonin reuptake inhibitor (SSRI), a 5-hydroxytryptamine (5-HT) receptor antagonist, a peroxisome proliferating activator receptor (PPAR) agonist, a calcineurin inhibitor, a gastrin-releasing peptide receptor antagonist, a p38 MAP kinase inhibitor, a keratinocyte growth factor (KGF), an HDAC inhibitor, a retinoid, a NFκB modulator, gabapentin, sucralfate, and naloxone.

The compounds can be formulated as pharmaceutical compositions. Such compositions can be administered orally, parenterally, by inhalation spray, rectally, vaginally, intradermally, transdermally, or topically in dosage unit formulations containing conventional nontoxic pharmaceutically acceptable carriers, adjuvants, and vehicles as desired. Topical administration may also involve the use of transdermal administration such as transdermal patches or iontophoresis devices. The term parenteral as used herein includes subcutaneous, intravenous, intramuscular, or intrasternal injection, or infusion techniques. Formulation of drugs is discussed in, for example, Hoover, John E., Remington's Pharmaceutical Sciences, Mack Publishing Co., Easton, Pa. (1975), and Liberman, H. A. and Lachman, L., Eds., Pharmaceutical Dosage Forms, Marcel Decker, New York, N. Y. (1980).

In one embodiment, the preparations for treatment of skin side effects resulting from the use of the EGFR, tyrosine kinase or MAPK/ERK inhibitor are generally aimed at providing a condition for increasing skin manageability. There are recognized categories of formulations for skin care compositions, including creams, ointments, gels, sprays, lotions, skin tonics, shampoos or mousses. Skin sprays are generally composed of aerosolized copolymers, such as polyvinylpyrrolidone, vinyl acetate and the like, and may also function as a setting lotion. Skin gel preparations are similar to sprays in composition, but are in gel and alcohol free form, and can coat the skin. Skin mousse is foam released under pressure from an aerosolized can. The HDAC inhibitor ingredient in a topical skin care composition according to the present invention is preferably present at a concentration of 0.00001 to 100.00% by weight relative to the total weight of the composition, or in a dosage of 1 to 1000 mg. A skin care composition for treating skin lesions according to the present invention may be formulated as a hydrophobic or hydrophilic cream, ointment, gel, emollient, spray, lotion, skin tonic, shampoo or mousse, suitably with additional ingredients suitable for use in skin care compositions of types known in the art, wherein such further ingredients can include petrolatum, waxes, lanolin, silicone, liposomes, vegetable, mineral oils, plasticizers, fragrances, preservatives, a penetration enhancing agent, a pH adjusting agent or other suitable ingredients for topical skin compositions. Such ingredients can moisturize skin, stabilize the active compound, increase drug-skin contact and local concentration, control drug slow release, and/or aid in decreasing skin breakage, preventing skin atrophy, fibrosis and infection, and promoting skin wound healing. The pH adjusting agent is provided to adjust the formulation pH in the range of about 3.0 to 13.0.

Described herein is pharmaceutical composition for use in treatment of skin side effects resulting from use of the EGFR, tyrosine kinase, VEGF, serine/threonine kinase, or MAPK/ERK inhibitor as described herein, comprising a composition providing at least an HDAC inhibitor, together with at least one or more other agents, including a cytokine, a cytokine inhibitor, an interleukin, an interleukin inhibitor, a growth factor, an angiogenic agent, an angiogenic inhibitor, an anti-neoplastic agent, an anti-inflammatory agent, a steroid, an immunosuppressive agent, a non-steroid anti-inflammation drug, an analgesic agent, an antihistamine, an anticholinergics, an antipruritic agent, an antibacterial agent, an antiviral agent, an antifungal agent, an antiparasitic agent, an anti-oxidant agent, retinoic acid, an anti-fibrogenic agent, a vasoactive agent, an adenosine receptor agonist, a vitamin, a leukotriene modifier, an interleukin antagonist, a chemokine antagonist, a mast cell inhibitor, an anti-IgE antibody, a selective serotonin reuptake inhibitor (SSRI), a 5-hydroxytryptamine (5-HT) receptor antagonist, a peroxisome proliferating activator receptor (PPAR) agonist, a calcineurin inhibitor, a gastrin-releasing peptide receptor antagonist, a p38 MAP kinase inhibitor, a keratinocyte growth factor (KGF), an HDAC inhibitor, a retinoid, a NFκB modulator, gabapentin, sucralfate and naloxone, in which active ingredients are present in free form or in the form of a pharmaceutically acceptable salt or solvate for systemically or topically simultaneous, separate or sequential use.

The mucocutaneous, ocular toxicities, or side effects include dermatological or epithelial side effects of papulopustular rash, dry skin, itching, dermatitis, hand foot syndrome, mucositis, loss of hair, increased growth of the eyelashes and facial hair, and/or brittle deformed nails and periungual swelling. The EGFR, tyrosine kinase, VEGF, serine/threonine kinase, or MAPK/ERK inhibitor may be an antibody targeted therapy agent, antibody fragment, targeted small-molecule chemical compound, low-molecular-weight tyrosine kinase inhibitor, peptide mimetic, anti-sense oligonucleotide (DNA and/or RNA), or ribozyme, which inhibits epidermal growth factor receptor (EGFR), MAPK(mitogen-activated protein kinase)/ERK (extracellular regulated kinase), vascular endothelial growth factor (VEGF), and/or PI3K (phosphatidylinositol 3-kinase)-Akt-mTOR pathways.

Suitable salts for the components to be employed according to the present subject matter are also those with inorganic cations, for example alkali metal salts, in particular sodium, potassium, or ammonium salts, alkaline earth metal salts such as, in particular, the magnesium or calcium salts, as well as salts with bi- or tetravalent cations, for example the zinc, aluminum, or zirconium salts. Also contemplated are salts with organic bases, such as dicyclohexylamine salts; methyl-D-glucamine; and salts with amino acids, such as arginine, lysine, histidine, glutamine and so forth. Also, the basic nitrogen-containing groups can be quaternized with such agents as lower alkyl halides, such as methyl, ethyl, propyl, and butyl chlorides, bromides, and iodides; dialkyl sulfates, such as dimethyl, diethyl, dibutyl, and diamyl sulfates; long chain halides, such as decyl, lauryl, myristyl, and stearyl chlorides, bromides, and iodides; aromatic halides, such as benzyl and phenethyl bromides; and others. Salt-forming agents, for example, low molecular weight alkylamines such as methylamine, ethylamine, or triethylamine can also be employed. Water or oil-soluble or dispersible products are thereby obtained.

The amount of active ingredient that can be combined with the carrier materials to produce a single dosage will vary depending upon the subject and the particular mode of administration. The dosage required will vary according to a number of factors known to those skilled in the art, including, but not limited to, the compound or compounds used, the species of subject, the size of the subject, and the severity of the associated disease condition. The compounds can be administered in a single dose, in multiple doses throughout a 24-hour period, or by continuous infusion. When administered by continuous infusion, the compounds can be supplied by methods well known in the art, such as, but not limited to, intravenous gravity drip, intravenous infusion pump, implantable infusion pump, or any topical routes. Length of treatment will vary depending on many factors, for example, the duration and severity of the skin or mucosa lesions. Treatment of the subject with the HDAC inhibitor or in combination with other agents may last until the mucocutaneous or ocular lesions disappear, or treatment will continue for the life of the subject.

In one embodiment for use in treating the mucosa lesions, a biocompatible polymer is selected so that when the biocompatible polymer is incorporated into the therapeutic composition, the viscosity of the therapeutic composition increases with increasing temperature in the vicinity of physiological temperature, which is typically about 37°C. In this way, the therapeutic composition can be administered as a lower viscosity flowable fluid medium at a cool temperature, and the viscosity of the therapeutic composition will increase as the therapeutic composition is warmed to physiological temperature. In one preferred embodiment for many applications when it is desirable for the therapeutic composition to exhibit reverse-thermal viscosity behavior, the therapeutic composition exhibits reverse-thermal viscosity behavior over at least some range of temperatures between 1 °C and the physiological temperature of the host (e.g., 37°C for a human host). Biocompatible polymers that may be used to make the therapeutic composition include, in general, a bioadhesive polymer, a cationic polymer, a viscous polymer gel, a hydrogel, a natural polymer, a polyoxyalkylene block copolymer, and a reverse-thermal gelation polymer. The therapeutic composition may include, in addition to the biocompatible polymer, a separate bioadhesive agent that enhances the bioadhesive properties of the therapeutic composition. The bioadhesive agent is frequently a second polymer having even greater bioadhesive properties.

The therapeutic composition can be further formulated with sucralfate in a thicker gel form that can be spooned into the mouth and swallowed to coat the whole GI tract. Depending on the area of delivery the pharmaceutical substance can also be formulated in different product forms. Some examples of possible product forms for administration of the therapeutic composition include an oral solution, bladder irrigation solution, gel, slurry, mouthwash, lozenge, tablet, film, patch, lollipop, spray, drops or suppository. For example, a gel formulated into a suppository would be one preferred product form for administration to treat mucosal surfaces of either the rectum or the vagina. A slurry or oral solution could be used for treatment of mucosal surfaces in the oral cavity, esophagus and/or GI tract.

### EXAMPLE

### Example 1 : Suppression of CCL2 by HADC inhibitor

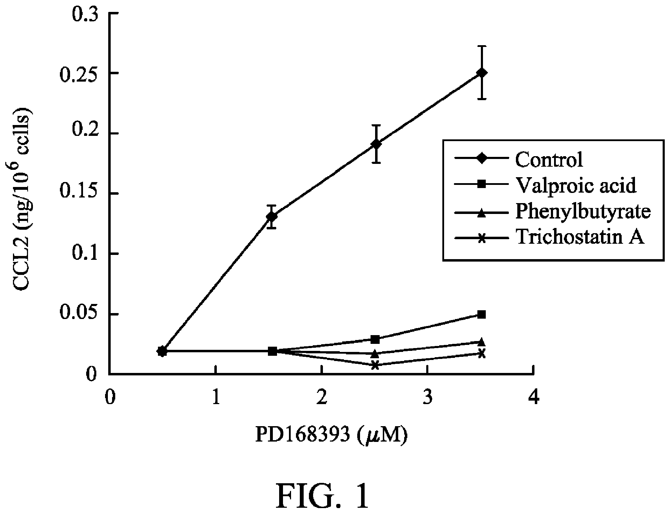

Because inhibition of EGFR pathways can cause skin reaction with increased epidermal levels of CCL2, ELISA was used to detect the CCL2 levels in the supernatant of keratinocyte cultures treated with or without the EGFR inhibitor PD 168393 and/or different HDAC inhibitors. The up-regulation of chemokine CCL2 levels by the 24-hour treatment of the EGFR inhibitor (PD168393) was suppressed in the supernactants from the keratinocytes that were pre-treated with different HDAC inhibitors (valproic acid (5 mM), phenylbutyrate (5 mM), and trichostatin A (5 nM)), respectively, for 2 hours (FIG. 1). Results are expressed as the mean of three independent experiments ±SD.

### Example 2: Preparation of different formulations of HDAC inhibitor for skin treatment

### A: Preparation of Gel of Phenylbutyrate

Part I: 10 g of Stabileze QM.RTM. and 380.561 g of deionized water were mixed in a beaker and heated at 70⁰C.
Part II: 5.739 g of sodium 4-phenylbutyrate (Merck), 0.125 g of methylparaben (Merck), 0.075 g of propylparaben (Merck), 83.5 g of 1,2-propandiol, and 20 g of 10% NaOH were mixed in a beaker and heated at 70⁰C.

The part II was slowly added into the part I and continually stirred at 400 rpm for 20 minutes to form a mixture. The mixture was cooled at room temperature.

### B: Preparation of Liposomal Formulation of Phenylbutyrate

In this liposomal formulation, egg phosphatidylcholine (EPC) and cholesterol were used in equi- or different-molar concentrations as primary lipid components. Various liposomes located with sodium 4-phenylbutyrate were obtained by varying the ration of lipid and phenylbutyrate. Liposomes were prepared by thin film hydration, sized by membrane extrusion, and physically evaluated.

### C: Preparation of Ointment of Trichostatin A

472.5 g of white petrolatum (Riedel-de Haen), 27 g of paraffin wax 50/52 (local supplier), and 0.5 g of trichostatin A (sigma) were mixed in a beaker and heated at 70⁰C to form a paste. The paste was stirred at 400 rpm for 1 hour, and then cooled at room temperature.

### D: Preparation of an Oleaginous Ointment of Trichostatin A

67.5 g of white petrolatum (Riedel-de Haen), 16 g of cetyl alcohol (Riedel-de Haen), 260.5 g of soft paraffin (Merck), 155.5 g of liquid paraffin (Merck), and 0.5 g of trichostatin A (sigma) were mixed in a beaker and heated at 70°C to form a paste. The paste was stirred at 400 rpm for 1 hour, and then cooled at room temperature.

### E: Preparation of Cream of Valproic Acid

Part I: 70 g of Tefose 63.RTM., 20 g of Superpolystate.RTM., 10 g of Coster 5000.RTM., 15 g of Myriyol 318.RTM., 15 g of Coster 5088.RTM., and 15 g of GMS SE. RTM. (all commercially available from local supplier) were mixed in a beaker and heated at 70°C.
Part II: 5.739 g of valproic acid (sigma), 0.125 g of methylparaben (Merck), 0.075 g of propylparaben (Merck), and 149.061 g of deionized water were mixed in a beaker and heated at 70⁰C.

The part II was slowly added into the part I and continually stirred at 400 rpm for 5 minutes to form a mixture. 2% Stabileze QM.RTM.D (prepared by dissolving 2 g of Stabileze QM.RTM. in 98 g of deionized water, heated and stirred at 70°C to form a paste, and cooled at room temperature) was added into the mixture and stirred for 5 minutes. The pH of the mixture was adjusted to 5.34 with 0.85% phosphoric acid (Merck), and stirred at 600 rpm for 20 minutes. The mixture was cooled at room temperature.

### Example 3 : Suppression of the EGFR inhibitor by the HDAC inhibitor in vivo

For the EGFR inhibitor-augmented skin reaction test, groups (n = 5, each) of BALB/c male mice weighing 22 ± 2 g received topical application on each side of each ear of 10 µL of the vehicle (DMSO/absolute ethanol 1/10) or solutions of PD168393 (4 mmol/L) 30 minutes before 10 µL of 0.5% 2,4-dinitrofluorobenzene (DNFB) irritation on the right ear of testing animals (Pastore S, et al. J Immunol 174:5047-5056, 2005).

To treat the EGFR inhibitor-augmented skin reaction, 2.5% 4-phenylbutyrate gel, or the placebo (gel base) was applied topically on the right ear 3 times at 3-hour interval before hand on day 0 and day 1. On day 1, 60 minutes after the first treatment of phenylbutyrate or placebo, PD168393 was applied topically 30 minutes before 0.5% DNFB irritation on the right ear. The second and third treatments of phenylbutyrate and placebo on day 1 were applied 1 hour and 3 hours after DNFB irritation. Ear swelling was measured with a Dyer model micrometer gauge at 0, 3, 6, 8, 24 and 48 hours after DNFB irritation. As a treatment control for comparison, the strong steroid dexamethasone (0.3 mg) was administered topically 60 minutes before and 15 minutes after DNFB irritation. The right and left ear thickness of each mouse was measured with a Dyer model micrometer gauge. Ear edema was calculated by subtracting the thickness of the left ear (normal control) from the right ear (treated ear).

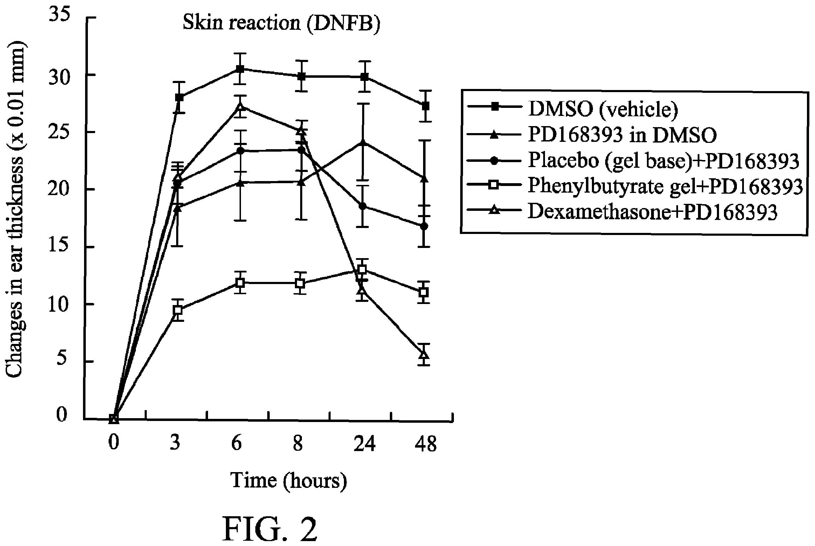

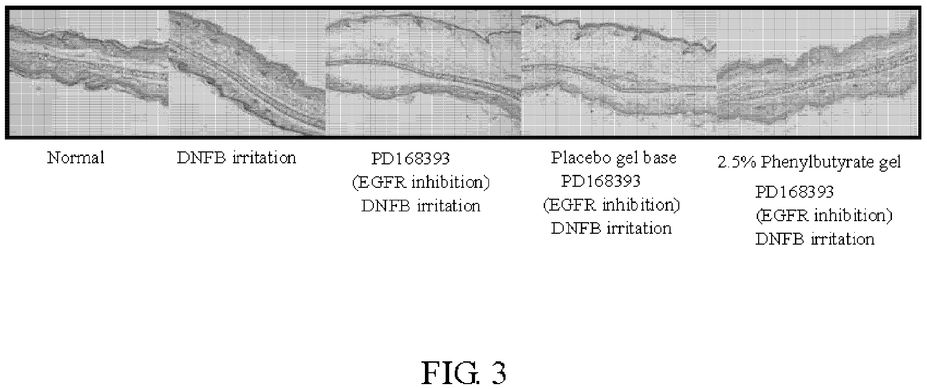

Referring to Figs 2 to 3, topical administration of 4 mM PD 168393 alone, DMSO alone, the placebo gel base only, or the 2.5% phenylbutyrate gel had no effect on ear thickness, and did not induce any change in normal skin histology. By contrast, 4 mM PD 168393 applied 30 minutes before DNFB irritation led to aggravation of the DNFB-induced skin response. However, the skin pre-treated with the 2.5% phenylbutyrate gel resulted in a significant reduction of the EGFR inhibitor-augmented ear swelling induced by DNFB irritation at 3, 6, 8, 24, and 48 hours after DNFB irritation as compared to the skin pre-treated with the placebo gel base (FIGs. 2 and 3). Although dexamethasone did not suppress the EGFR inhibitor- augmented skin reaction with peak at 3, 6, and 8 hours after DNFB irritation, it did show a significantly suppressive effect on the skin swelling 24 hours after DNFB irritation. The suppressive extent by dexamethasone on the skin swelling at 24 hours after DNFB irritation was larger than that by phenylbutyrate. Therefore, the results suggested that dexamethasone is not effective in suppression of the EGFR inhibitor-augmented skin reaction, and the 2.5% phenylbutyrate gel appears to have therapeutic benefit on the dermatological side effects augmented by inhibition of EGFR.

While the invention has been described by way of example and in terms of the preferred embodiments, it is to be understood that the invention is not limited to the disclosed embodiments. To the contrary, it is intended to cover various modifications and similar arrangements (as would be apparent to those skilled in the art). Therefore, the scope of the appended claims should be accorded the broadest interpretation so as to encompass all such modifications and similar arrangements.

## Claims

1. A pharmaceutical composition comprising a therapeutically effective amount of a histone deacetylase (HDAC) inhibitor and/or a pharmaceutically acceptable salt or solvate thereof for use in treatment of mucocutaneous or ocular toxicities or side effects resulting from blocking of cell growth and survival signal transduction pathways caused by an EGFR (epidermal growth factor receptor) inhibitor, wherein the HDAC inhibitor comprises valproic acid, phenylbutyrate or trichostatin A.

2. The composition for use as claimed in claim 1, wherein the pharmaceutical composition is non-orally administered.

3. The composition for use as claimed in claim 1, wherein in the pharmaceutical composition an amount of the HDAC inhibitor of from 0.00001% to 100% by weight of the pharmaceutical composition is present.

4. The composition for use claimed in claim 1, wherein the pharmaceutical composition is a cream, an ointment, a gel, a paste, a powder, an aqueous solution, a spray, a suspension, a dispersion, a slave, a lotion, a patch, a suppository, a liposome formation, a mouth wash, an enema, an injection solution, an eye drop, an ear drop, a drip infusion, a microcapsule, a nanocapsule, an occlusive skin conditioning agent, a biocompatible polymer, or an agent to prolong retention and sustain action of the HDAC inhibitor in the tissue.

5. The composition for use as claimed in claim 1, wherein the pharmaceutical composition further comprises a penetration enhancing agent, or a pH adjusting agent to provide a pH in the range of approximately 3.0 to 13.0.

6. The composition for use as claimed in claim 1, wherein the mucocutaneous or ocular toxicities, or side effects comprise dermatological or epithelial side effects including papulopustular rash, dry skin, itching, dermatitis, hand foot syndrome, mucositis, loss of hair, increased growth of the eyelashes and facial hair, and/or brittle deformed nails and periungual swelling.

## Patentansprüche

1. Pharmazeutische Zusammensetzung, umfassend eine therapeutisch wirksame Menge eines Histondeacetylase (HDAC)-Inhibitors und/oder eines pharmazeutisch verträglichen Salzes oder Solvates davon, zur Verwendung in der Behandlung von mukokutanen oder okularen Toxizitäten oder Nebenwirkungen, die aus dem Blockieren von Zellwachstums- und Überlebenssignaltransduktionswegen resultieren, verursacht durch einen EGFR (epidermaler Wachstumsfaktor-Rezeptor)-Inhibitor, wobei der HDAC-Inhibitor Valproinsäure, Phenylbutyrat oder Trichostatin A umfasst.

2. Zusammensetzung zur Verwendung wie in Anspruch 1 beansprucht, wobei die pharmazeutische Zusammensetzung nicht oral verabreicht wird.

3. Zusammensetzung zur Verwendung wie in Anspruch 1 beansprucht, wobei in der pharmazeutischen Zusammensetzung eine Menge des HDAC-Inhibitors von 0,00001 Gew.-% bis 100 Gcw.-% der pharmazeutischen Zusammensetzung vorhanden ist.

4. Zusammensetzung zur Verwendung wie in Anspruch 1 beansprucht, wobei die pharmazeutische Zusammensetzung eine Creme, eine Salbe, ein Gel, eine Paste, ein Pulver, eine wässrige Lösung, ein Spray, eine Suspension, eine Dispersion, eine Slave, eine Lotion, ein Pflaster, ein Zäpfchen, eine Liposomen-Formulierung, eine Mundspülung, ein Einlauf, eine Injektionslösung, ein Augentropfen, ein Ohrentropfen, eine Tropfinfusion, eine Mikrokapsel, eine Nanokapsel, ein okklusives Hautkonditioniermittel, ein biokompatibles Polymer oder ein Mittel zur Verlängerung der Retention und Aufrechterhaltung der Wirkung des HDAC-Inhibitors in dem Gewebe ist.

5. Zusammensetzung zur Verwendung wie in Anspruch 1 beansprucht, wobei die pharmazeutische Zusammensetzung ferner ein penetrationssteigerndes Mittel oder ein pH-Wert-einstellendes Mittel zur Bereitstellung eines pH-Wertes im Bereich von ungefähr 3,0 bis 13,0 umfasst.

6. Zusammensetzung zur Verwendung wie in Anspruch 1 beansprucht, wobei die mukokutanen oder okularen Toxizitäten oder Nebenwirkungen dermatologische oder epitheliale Nebenwirkungen, einschlicßlich papulopustulösem Exanthern, trockener Haut, Juckreiz, Dermatitis, Hand-Fuß-Syndrom, Mukositis, Haarverlust, erhöhtem Wachstum der Wimpern und Gesichtsbehaarung und/oder brüchiger deformierter Nägel und periungualer Schwellung, umfassen.

## Revendications

1. Composition pharmaceutique comprenant une quantité thérapeutiquement efficace d'un inhibiteur de l'histone désacétylase (HDAC) et/ou d'un sel ou solvate pharmaceutiquement acceptable de celui-ci pour une utilisation dans le traitement de toxicités mucocutanées ou oculaires ou des effets secondaires résultant du blocage de la croissance cellulaire et des voies de transduction de signal de survie provoqués par un inhibiteur de l'EGFR (récepteur de facteur de croissance épidermique), dans laquelle l'inhibiteur de l'HDAC comprend de l'acide valproïque, du phénylbutyrate ou de la trichostatine A.

2. Composition pour une utilisation selon la revendication 1, dans laquelle la composition pharmaceutique est administrée par voie non orale.

3. Composition pour une utilisation selon la revendication 1, dans laquelle, dans la composition pharmaceutique, une quantité de l'inhibiteur de HDAC de 0,00001 % à 100 % en poids de la composition pharmaceutique est présente.

4. Composition pour une utilisation selon la revendication 1, dans laquelle la composition pharmaceutique est une crème, une pommade, un gel, une pâte, une poudre, une solution aqueuse, un spray, une suspension, une dispersion, un baume, une lotion, un patch, un suppositoire, une formation de liposome, un bain de bouche, un lavement, une solution pour injection, des gouttes oculaires, des gouttes auriculaires, une perfusion par goutte à goutte, une microcapsule, une nanocapsule, un agent de conditionnement cutané occlusif, un polymère biocompatible ou un agent pour prolonger la rétention et soutenir l'action de l'inhibiteur de HDAC dans le tissu.

5. Composition pour une utilisation selon la revendication 1, dans laquelle la composition pharmaceutique comprend en outre un agent de renforcement de la pénétration, ou un agent d'ajustement du pH pour donner un pH dans la plage d'environ 3,0 à 13,0.

6. Composition pour une utilisation selon la revendication 1, dans laquelle les toxicités mucocutanées ou oculaires, ou les effets secondaires comprennent des effets secondaires dermatologiques ou épithéliaux incluant une éruption papulo-pustulaire, une sécheresse cutanée, un prurit, une dermatite, un syndrome main pied, une mucosite, une alopécie, une croissance accrue des cils et de la pilosité faciale et/ou des ongles cassants déformés et un gonflement péri-unguéale.
